Europäisches Patentamt

European Patent Office

Office européen des brevets

(11) Veröffentlichungsnummer: **0 192 823**
**B1**

(12) **EUROPÄISCHE PATENTSCHRIFT**

(45) Veröffentlichungstag der Patentschrift:
**14.06.89**

(51) Int. Cl.⁴: **G 01 N 27/46, A 61 B 5/00**

(21) Anmeldenummer: **85114418.8**

(22) Anmeldetag: **13.11.85**

(54) Verfahren und Vorrichtung zur Kalibrierung von Messwertaufnehmern zur Bestimmung von Gaskonzentrationen.

(30) Priorität: **26.02.85 DE 3506731**

(43) Veröffentlichungstag der Anmeldung:
**03.09.86 Patentblatt 86/36**

(45) Bekanntmachung des Hinweises auf die Patenterteilung:
**14.06.89 Patentblatt 89/24**

(84) Benannte Vertragsstaaten:
**CH DE FR GB LI NL**

(56) Entgegenhaltungen:
**EP-A- 0 031 149**
**EP-A- 0 074 498**
**DE-A- 2 645 736**
**DE-A- 2 841 050**
**GB-A- 2 140 563**

(73) Patentinhaber: **Drägerwerk Aktiengesellschaft,
Moislinger Allee 53-55, D-2400 Lübeck 1 (DE)**

(72) Erfinder: **Rogge, Bernd, Dr. Dipl.-Chem.,
Gorch-Fock-Ring 40, D-2409 Scharbeutz (DE)**
Erfinder: **Kaross, Klaus, Wesloer Strasse 35,
D-2400 Lübeck 16 (DE)**
Erfinder: **Busack, Hans-Jürgen, Ing. grad., Niendorfer
Hauptstrasse 35, D-2400 Lübeck 1 (DE)**
Erfinder: **Lungu, Mihail, Dr. Dipl.-Chem., Eichenweg 17,
D-2067 Reinfeld (DE)**

## Beschreibung

Die Erfindung betrifft ein Verfahren zur Kalibrierung von Messwertaufnehmern zur Bestimmung von Gaskonzentrationen, insbesondere zur transcutanen Messung von Gasen im Blut, bei welchem die Messkammer an ihrer Messfläche durch eine Auflage einer bekannten Konzentration des nachzuweisenden Gases ausgesetzt wird. Ausserdem wird eine Auflage zur Durchführung des Kalibrierverfahrens angegeben.

Ein derartiges Verfahren ist aus der DE-OS 26 45 736 bekanntgeworden.

Dort ist zur Kalibrierung eines Messwertaufnehmers mit einer geeigneten Kalibrierlösung, beispielsweise Wasser oder Natriumsulfit, eine der Messfläche des Messwertaufnehmers angepasste Auflage vorgesehen. Sie weist ein Kalibriervolumen auf, welches exakt über der Messfläche positioniert werden muss. In dieses Kalibriervolumen wird die Kalibrierlösung eingebracht, darin gehalten und wieder entfernt. Die Auflage besteht beispielweise aus einer Klebeschicht und einer darüber befindlichen Deckschicht, wobei in der Klebeschicht eine filzartige Einlage vorgesehen sein kann, welche durch geeignete Mittel zentrisch über die Messfläche angeordnet wird, um die Kalibrierlösung aufzunehmen.

Typischerweise werden zur Kalibrierung der bekannten Messwertaufnehmer zwei verschiedene Kalibrierlösungen gewählt, die im Falle der Kalibrierung eines Messwertaufnehmers zur transcutanen Bestimmung des Blutsauerstoffgehaltes aus reinem Wasser und einer Natriumsulfitlösung in flüssiger oder auch pastenförmiger Ausführung bestehen. In beiden Fällen muss dafür gesorgt werden, dass mit Hilfe der dafür vorgesehenen Lokalisierringe die entsprechenden Kalibriervolumina genau über der Messfläche angeordnet sind. Es ist darauf zu achten, dass in den in die Kalibriervolumina eingebrachten Kalibrierlösungen keine Luftblasen entstehen, oder dass die Messfläche stets während des ganzen Kalibriervorganges die Messfläche vollständig überdeckt. Aus diesen Forderungen resultiert eine vorgeschriebene Lagerung des Messwertaufnehmers während des Kalibriervorganges. So können bespielsweise Messwertaufnehmer zur transcutanen Bestimmung des Blutsauerstoffgehaltes nach dem bekannten Verfahren nur in waagerechter Position kalibriert werden.

Auch die Zubereitung der Kalibrierlösungen erfordert weitere Handhabungsschritte, welche vor der eigentlichen Kalibrierung durchgeführt werden müssen. Es sind nämlich die notwendigen Kalibrierlösungen zunächst nach bestimmten Vorschriften anzusetzen, wobei die Haltbarkeit der angesetzten Lösungen begrenzt ist. So ist beispielsweise für die Herstellung einer geeigneten Natriumsulfitlösung zunächst Natriumsulfit in reinem Wasser in einer bestimmten Menge zu lösen, und mittels weiterer Vorrichtungen, wie zum Beispiel Pipetten, in Tropfenform in das Kalibriervolumen einzubringen. Die einmal angesetzte Natriumsulfitlösung ist über längere Zeiträume nicht haltbar, sondern sie muss nach typischerweise einem Monat verworfen und neu angesetzt werden.

Nach erfolgter Kalibrierung sind sowohl die Kalibrierlösung als auch die der Zentrierung des Kalibriervolumens dienenden Halteringe zu entfernen und die Messfläche auf besonders sorgfältige Weise von Rückständen der Kalibrierlösung zu befreien.

Es ist zwar aus EP-A-31 149 bekannt, die Brauchbarkeit von Kalibrierlösungen, insbesondere Natriumsulfit, anhand der Verschiebung des pH-Wertes mittels eines dazu geeigneten Farbindikators anzuzeigen und bei Erreichen eines gewissen Grenzwertes für den pH-Wert anhand des Farbumschlages, beispielsweise von blau nach gelb, die Kalibrierlösung als unbrauchbar zu verwerfen, jedoch erspart sie dem Benutzer nicht, bei unbrauchbar gewordener Kalibrierlösung eine neue Lösung anzusetzen, welche wiederum nur begrenzt haltbar ist.

Des weiteren hat das versehentliche Vorhandensein auch nur geringer Mengen von schwachen Säuren oder Laugen in der Kalibrierlösung eine unkontrollierte Verschiebung des pH-Wertes zur Folge, wodurch eine direkte Korrelation zwischen einem Farbumschlag und der Brauchbarkeit bzw. Unbrauchbarkeit der Kalibrierlösung nicht mehr gegeben ist.

Die Erfindung geht von der Aufgabe aus, ein Verfahren der bekannten Art derart zu verbessern, dass die gebrauchsfertige Kalibrierlösung lediglich in der für eine einzige Kalibrierung erforderlichen geringen Menge unmittelbar vor dem Kalibriervorgang erzeugt wird, und dass die Kalibrierlösung mittels der Auflage ohne weitere Hilfsmittel auf die Messfläche gebracht werden kann und ohne dass ein Kalibriervolumen in genauer Zentrierung über der Messfläche angeordnet sein muss.

Die Lösung wird durch die Merkmale des Anspruchs 1 erreicht.

Die Haltbarkeit der mit der Substanz versehenen Auflage ist im Vergleich zu der kurzzeitstabilen Kalibrierlösung unbegrenzt. Durch das unmittelbare Aufbringen der Auflage auf die Messfläche liegt die zur Kalibrierung notwendige Substanz flächig auf der Messfläche auf, ohne dass zusätzliche Hilfsmittel zur Aufbringung oder Zentrierung erforderlich sind.

Zweckmässigerweise ist die Auflage in an sich bekannter Weise saugfähig zu gestalten und mit löslichen Substanzen zu versehen, und sie vor dem Aufbringen auf die Messfläche mit einem Lösungsmittel zu tränken. Da die Auflage nach ihrem Tränken mit Lösungsmittel einen geschlossenen, grossflächigen Film an Kalibrierlösung bildet, wird wegen ihrer Saugfähigkeit und Porosität eine für die Durchführung des Kalibriervorganges genügende Menge an Kalibrierlösung in ihr gespeichert. Durch die getränkte Auflage wird über der gesamten Messfläche ein Nässefilm hergestellt, welcher diese ohne Bildung von Luftblasen oder ähnlichen Störstellen auch auf hydrophoben Membranen, wie sie in der Regel als Abschluss der Messfläche benutzt werden, vollständig mit der Kalibrierlösung bedeckt. Durch die Ausbildung des genannten Nässefilmes bleibt die Auflage auf der Messfläche infolge der Adhäsionskräfte in jeder erdenklichen Lage des Messwertaufnehmers haften, so dass der Kalibriervorgang in jeder beliebigen Stellung des Messwertaufnehmers durchgeführt werden kann. Ist der Kalibriervorgang beendet, wird die Auflage durch gleitendes Abziehen von der Messfläche entfernt, ohne diese zu beschädigen und wobei gleichzeitig sämtliche Rückstände der Kalibrierlösung mit abgewischt werden. Ein zusätzlicher nachträglicher Reinigungsvorgang entfällt.

Soll eine Null-Kalibrierung durchgeführt werden, ist die Auflage vorteilhafterweise mit Natriumsulfit zu imprägnieren. Diese Imprägnierung kann zweckmässigerweise aus einer 70% bis 100% gesättigten Natriumsulfitlösung erfolgen, wobei nach Verdampfung des Lösungsmittels Natriumsulfit in fester Form in der Auflage vorliegt. Natriumsulfit wirkt bekanntermassen als ein Sauerstoff verbrauchendes Agens, so dass der Luftsauerstoff durch die getränkte Auflage hindurch nicht an die Messfläche gelangt.

Als zusätzlicher Kalibrierschritt wird neben der Null-Kalibrierung eine Kalibrierung mit Luftsauerstoff vorgenommen. Da der in der Luft enthaltene Wasserdampf zu einer Verfälschung des Kalibrierwertes führen kann, ist es wünschenswert, trockenes Kalibriergas zu verwenden. Durch eine zusätzliche Imprägnierung oder Beschichtung mit Silicagel als einer weiteren Substanz wird bei Anwendung der trockenen Auflage der herandiffundierende Wasserdampf aus der Luft zurückgehalten. Die Berücksichtigung einer zusätzlichen Wasserdampfkorrektur ist somit nicht mehr erforderlich.

Soll eine Kalibrierung eines Messwertaufnehmers zur transcutanen Bestimmung von $CO_2$ durchgeführt werden, wird die Auflage zweckmässigerweise mit einer carbonathaltigen Substanz versehen.

Um die imprägnierte Substanz zur Freigabe des Kalibriergases zu aktivieren, kann Wasser benutzt werden, an welches im Gegensatz zu den bekannten Kalibrierlösungen keine besonderen Reinheitsforderungen gestellt werden. Es können gegebenenfalls sogar schwache Säuren oder Basen als Lösungsmittel verwendet werden, ohne dass die Wirksamkeit der Kalibrierlösung eingeengt wird.

Als Auflage zur Durchführung des Verfahrens zur Kalibrierung von Messwertaufnehmern zur Bestimmung von Gaskonzentrationen ist in besonders einfacher Weise ein Streifen geeignet, der aus porösem, durch das Lösungsmittel benetzbarem Material, wie Papier, besteht, welcher mit einer Substanz versehen ist, die nach dem Benetzen des Papierstreifens mit einem geeigneten Lösungsmittel die zur Kalibrierung benötigte bekannte Gaskonzentration bereitstellt. Benutzt man gemäss den Verfahrensschritten dazu beispielsweise Natriumsulfit als eine imprägnierte Substanz und löst diese Substanz z.B. mit Wasser auf, erhält man einen Papierstreifen, welcher, auf die Messfläche des Messwertaufnehmers gebracht, eine sauerstofffreie Gaskonzentration über der Messfläche erzeugt. Dadurch erfolgt in besonders einfacher Weise eine Null-Kalibrierung des Messwertaufnehmers.

Zur leichteren Handhabung kann der Papierstreifen teilweise mit einem Überzug versehen sein, an welchem dieser zum Auflegen auf die Messfläche des Messwertaufnehmers angefasst werden kann.

Der Papierstreifen kann in besonders günstiger Weise aus mehreren benachbarten Schichten aufgebaut sein, die entweder unmittelbar aufeinanderliegen oder auch, je nach Erfordernis, durch eine geeignete Zwischenlage, z.B. zur Erhöhung der Haftung der Schichten aneinander, miteinander verbunden sind. Eine erste Schicht ist beispielsweise mit Natriumsulfit, eine zweite Schicht mit Silicagel zur Zurückhaltung von Wasserdampf imprägniert.

Zur Durchführung mehrerer hintereinanderfolgender Kalibriervorgänge mit unterschiedlichen Gaskonzentrationen kann in weiterer Ausgestaltung der Erfindung der Papierstreifen in mehrere Zonen aufgeteilt sein, welche mit unterschiedlichen Substanzen zur Bereitstellung der benötigten bekannten Gaskonzentrationen imprägniert sind.

So kann beispielsweise eine erste Zone zur Null-Kalibrierung eines Messwertaufnehmers für die Sauerstoffmessung mit Natriumsulfit, und eine zweite Zone zur Erzeugung einer bekannten $CO_2$-Konzentration für eine Kalibrierung eines Messwertaufnehmers zur $CO_2$-Messung mit einer carbonathaltigen Substanz imprägniert sein. Beide Zonen können vor der Durchführung der Kalibrierung mit ein und demselben Lösungsmittel, zum Beispiel Wasser, getränkt werden, um die zur Kalibrierung notwendige bekannte Gaskonzentration bereitzustellen.

Um ein Vermischen der getränkten, mit unterschiedlichen Substanzen imprägnierten Zonen zu verhindern, ist es vorteilhaft, diese durch ein Zwischenstück voneinander zu trennen, welches beispielsweise von dem Lösungsmittel nicht benetzbar ist und sich gegenüber den verwendeten Substanzen indifferent verhält.

Zur Durchführung des Verfahrens hat es sich als besonders günstig erwiesen, die Papierstreifen nicht dünner als etwa 0,2 mm bis 1 mm auszuführen. Damit wird sichergestellt, dass beispielsweise bei der Durchführung einer Null-Kalibrierung mittels imprägnierten und gelösten Natriumsulfits eine Diffusion von Sauerstoff aus der Atmosphäre durch den getränkten Papierstreifen zur Messfläche des Messwertaufnehmers während des Kalibriervorganges verhindert wird.

Es ist ohne weiteres ersichtlich, dass ein derartiger Papierstreifen in beliebiger, bekannter Weise zur Erleichterung der Handhabung konfektioniert werden kann. So kann beispielsweise zur Entnahme eines zur Kalibrierung vorgesehenen Kalibrierstreifens dieser auf einer Rolle zu mehreren hintereinanderliegenden Einzelstreifen aufgewikkelt sein, welche ihrerseits durch eine geeignete Perforation leicht voneinander trennbar sind.

Die Ausgestaltung der Papierstreifen kann den einzelnen Formen der Messflächen der Messwertaufnehmer durch geeignete Zuschnitte angepasst werden.

Weiterhin kann für grössere Messwertaufnehmer, welche eine höhere Gasmenge einer bekannten Gaskonzentration zur Kalibrierung benötigen, der Papierstreifen in dickerer und grossflächiger Ausführung vorgesehen sein, um die notwendige Substanzmenge aufbringen und mit dem geeigneten Lösungsmittel lösen zu können.

Im folgenden wird die Erfindung anhand einer schematischen Zeichnung beispielhaft dargestellt und erläutert.

Es zeigt:

Fig. 1: die Ansicht der Auflage

Fig. 2: einen Schnitt durch die Auflage

Fig. 3: eine geschnittene Auflage auf der Messfläche des Messwertaufnehmers.

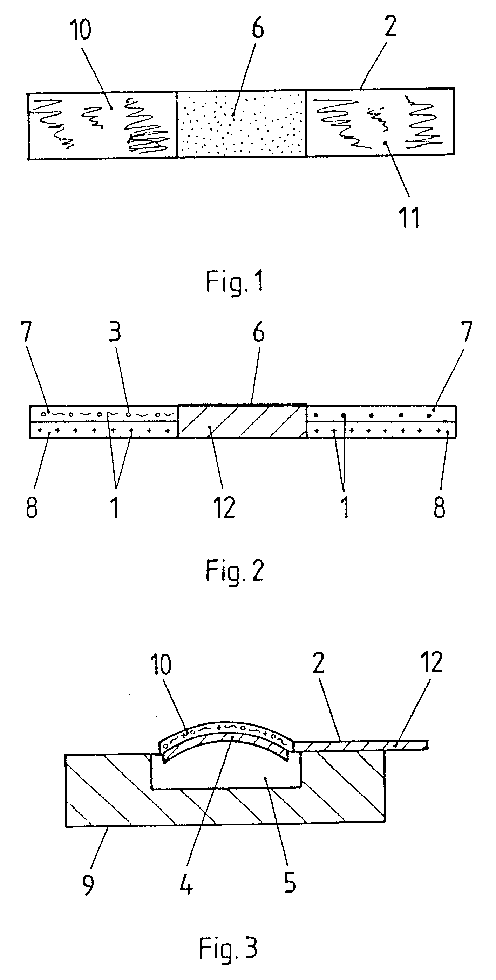

In Figur 1 ist die Auflage 2 dargestellt, welche aus zwei imprägnierten Zonen 10, 11 und einem zwischen den imprägnierten Zonen angeordneten hydrophoben Zwischenstück 12 besteht, wobei das Zwischenstück 12 mit einem Überzug 6 zur leichteren Handhabung der als Papierstreifen ausgebildeten Auflage 2 dient. Ein zur Durchführung der $O_2$-Null-Kalibrierung vorbereiteter Papierstreifen 2 ist in Figur 2 im Schnitt dargestellt. Jede Zone 10, 11 ist in zwei Schichten 7, 8 aufgeteilt. Dabei enthält die Schicht 7 der Zone 10 eine Imprägnierung aus Natriumsulfit (dargestellt durch Schlangenlinien), welches in einem Lösungsmittel 3 (dargestellt durch offene Kreise) gelöst ist. Die Schicht 8 ist in beiden Zonen 10, 11 mit Silicagel (dargestellt durch Kreuze) als Substanz 1 imprägniert. Die Schicht 7 der Zone 11 enthält eine (durch Punkte dargestellte) carbonathaltige Substanz 1. Das Zwischenstück 12 ist zwischen den beiden Zonen 10 und 11 angeordnet und verhindert ein Vermischen der gelösten Substanzen 1 aus den beiden Zonen 10 und 11. Figur 3 zeigt einen Papierstreifen 2, dessen Zone 10 auf der Messfläche 4 der Messkammer 5 eines Messwertaufnehmers 9 aufgelegt ist. Die Zone 10 besteht aus nur einer Schicht, welche mit der Imprägnierung aus Natriumsulfit zusätzlich eine Imprägnierung aus Silicagel enthält. Das als Endstück ausgebildete Zwischenstück 12 befindet sich ausserhalb des Bereiches der Messfläche 4 und dient zum Auflegen und Abziehen des Papierstreifens 2 auf und von der Messfläche 4.

Zur Durchführung einer Kalibrierung mit getrocknetem Luftsauerstoff wird der trockene Papierstreifen 2 über die Messfläche 4 gelegt, so dass der Sauerstoff der Luft durch die Schicht zur Messfläche 4 gelangt. Der Wasserdampf wird durch das Silicagel zurückgehalten.

Bei anschliessender Null-Kalibrierung wird die Schicht mit Wasser getränkt, wodurch das Natriumsulfit gelöst wird und den durch die Schicht diffundierenden Luftsauerstoff bindet.

## Patentansprüche

1. Verfahren zur Kalibrierung von Messwertaufnehmern (9) zur Bestimmung von Gaskonzentrationen, insbesondere zur transcutanen Messung von Gasen im Blut, bei welchem eine Messkammer (5) an ihrer Messfläche (4) durch eine Auflage (2) einer bekannten Konzentration des nachzuweisenden Gases ausgesetzt wird, dadurch gekennzeichnet, dass eine mit löslichen Substanzen (1) versehene Auflage (2) – wobei diese löslichen Substanzen (1) zur Bildung der bekannten Konzentrationen des zu bestimmenden Gases notwendig sind – mit einem Lösungsmittel (3) getränkt und danach unmittelbar auf die Messfläche (4) der Messkammer (5), diese abdeckend, aufgebracht wird.

2. Verfahren nach Anspruch 1, dadurch gekennzeichnet, dass die Auflage (2) mit Silicagel als einer weiteren Substanz zur Zurückhaltung von Wasserdampf versehen ist und dass vor der Tränkung der Auflage (2) mit einem Lösungsmittel (3) eine Luftkalibrierung durchgeführt wird.

3. Verfahren nach Anspruch 1, dadurch gekennzeichnet, dass die Auflage (2) mit Natriumsulfit als Substanz (1) versehen und zur Bildung einer $O_2$-Null-Kalibrierlösung mit Wasser als Lösungsmittel (3) getränkt wird.

4. Verfahren nach Anspruch 1, dadurch gekennzeichnet, dass die Auflage (2) mit Natriumsulfit aus einer 70% bis 100% gesättigten Natriumsulfitlösung versehen wird.

5. Verfahren nach Anspruch 1, dadurch gekennzeichnet, dass die Auflage (2) mit einer carbonathaltigen Substanz (1) versehen, und mit Wasser als Lösungsmittel (3) zur Bildung einer $CO_2$-Kalibrierlösung bekannter $CO_2$-Konzentration getränkt wird.

6. Auflage zur Durchführung einer Kalibrierung eines Messwertaufnehmers (9) zur Bestimmung von Gaskonzentrationen, insbesondere zur transcutanen Messung von Gasen im Blut, welche mit einer Substanz (1) zur Abgabe einer bekannten Konzentration des nachzuweisenden Gases versehen ist und über die Messfläche (4) des Messwertaufnehmers (9) angeordnet ist, dadurch gekennzeichnet, dass ein poröser, mit löslichen Substanzen (1) versehener Streifen (2) vorgesehen ist, der zur Belieferung der bekannten Konzentration des zu bestimmenden Gases mit einem die Substanzen (1) lösenden Lösungsmittel (3) benetzbar ist.

7. Auflage nach Anspruch 6, dadurch gekennzeichnet, dass der Streifen (2) teilweise mit einem Überzug (6) versehen ist.

8. Auflage nach Anspruch 6, dadurch gekennzeichnet, dass der Streifen (2) mehrere, mit unterschiedlichen Substanzen (1) versehene benachbarte Schichten (7, 8) aufweist.

9. Auflage nach Anspruch 8, dadurch gekennzeichnet, dass eine erste Schicht (7) zur Belieferung mit einer $O_2$-Null-Konzentration mit Natriumsulfit und eine zweite Schicht (8) zur Zurückhaltung von Wasserdampf aus der Umgebungsluft und Belieferung einer Luftsauerstoff-Konzentration mit Silicagel versehen ist.

10. Auflage nach Anspruch 6, dadurch gekennzeichnet, dass der Streifen (2) in mehrere mit unterschiedlichen Substanzen (1) versehene Zonen (10, 11) aufgeteilt ist.

11. Auflage nach Anspruch 10, dadurch gekennzeichnet, dass eine erste Zone (10) zur Bildung einer Null-Kalibrierlösung mit Natriumsulfit und eine zweite Zone (11) zur Bildung einer bekannten $CO_2$-Konzentration mit einer carbonhaltigen Substanz (1) versehen ist.

12. Auflage nach Anspruch 10, dadurch gekennzeichnet, dass die Zonen (10, 11) jeweils durch ein Zwischenstück (12) voneinander getrennt sind.

13. Auflage nach einem der Ansprüche 6 bis 12, dadurch gekennzeichnet, dass der Streifen (2) eine Dicke von etwa 0,2 mm bis 1 mm besitzt.

## Revendications

1. Procédé d'étalonnage de transducteurs (9) pour la détermination de concentrations de gaz, notamment pour la mesure transcutanée de gaz dans le sang, selon lequel une chambre de mesure (5) est exposée sur sa face de mesure (4), à l'aide d'un support (2), à une concentration connue du gaz à mesurer, caractérisé en ce qu'un support (2) contenant de substances solubles (1) – ces substances solubles (1) étant nécessaires pour former les concentrations connues du gaz à mesurer – est imprégnée d'un solvant (3), puis immédiatement appliquée sur la face de mesure (4) de la chambre de mesure (5), en la recouvrant.

2. Procédé selon la revendication 1, caractérisé en ce que le support (2) contient du silicagel comme autre substance pour retenir la vapeur d'eau, et en ce qu'un étalonnage à l'air est réalisé avant d'imprégner le support (2) d'un solvant (3).

3. Procédé selon la revendication 1, caractérisé en ce que le support (2) contient du sulfite de sodium comme substance (1), et est imprégnée d'eau comme solvant (3) pour former une solution d'étalonnage à zéro ne contenant pas d'$O_2$.

4. Procédé selon la revendication 1, caractérisé en ce que le support (2) contient du sulfite de sodium provenant d'une solution de sulfite de sodium saturée à une valeur comprise entre 70% et 100%.

5. Procédé selon la revendication 1, caractérisé en ce que le support (2) contient une substance (1) comprenant du carbonate, et est imprégnée d'eau comme solvant (3) pour former une solution d'étalonnage en $CO_2$ à concentration de $CO_2$ connue.

6. Support pour réaliser l'étalonnage d'un transducteur (9) pour la détermination de concentrations de gaz, notamment pour la mesure transcutanée de gaz dans le sang, lequel support contient une substance (1) pour délivrer une concentration connue du gaz à mesurer, et est disposé au-dessus de la face de mesure (4) du transducteur (9), caractérisé en ce qu'il est prévu une bande poreuse (2) contenant de substances solubles (1) qui, afin de délivrer la concentration connue du gaz à mesurer, est imprégnable par un solvant (3) qui dissout les substances (1).

7. Support selon la revendication 6, caractérisé en ce que la bande (2) est partiellement munie d'un revêtement (6).

8. Support selon la revendication 6, caractérisé en ce que la bande (2) présente plusieurs couches (7, 8) voisines, contenant des substances (1) différentes.

9. Support selon la revendication 8, caractérisé en ce qu'une première couche (7) contient du sulfite de sodium pour délivrer une concentration nulle en $O_2$, et en ce qu'une deuxième couche (8) contient du silicagel pour retenir la vapeur d'eau provenant de l'air ambiant et délivrer une concentration d'oxygène de l'air.

10. Support selon la revendication 6, caractérisé en ce que la bande (2) est divisée en plusieurs zones (10, 11) contenant respectivement des substances (1) différentes.

11. Support selon la revendication 10, caractérisé en ce qu'une première zone (10) contient de sulfite de sodium pour former une solution d'étalonnage à zéro, et en ce qu'une deuxième zone (11) contient une substance (1) comprenant du carbone pour former une concentration de $CO_2$ connue.

12. Support selon la revendication 10, caractérisé en ce que les zones (10, 11) sont séparées entre elles par un élément intermédiaire (12).

13. Support selon l'une des revendications 6 à 12, caractérisé en ce que la bande (2) présente une épaisseur d'environ 0,2 mm à 1 mm.

## Claims

1. Method for the calibration of measuring apparatuses (9) for determining gas concentrations, in particular for the transcutaneous measuring of gases present in the blood, in which method a measuring chamber (5) is interrupted on its measuring face (4) by a coating (2) of a known concentration of the gas which is to be identified, characterised in that a coating (2) provided with soluble substances (1) – these soluble substances (1) being necessary for the formation of the known concentrations of the gas to be determined – is impregnated with a solvent (3) and then applied directly on to the measuring face (4) of the measuring chamber (5), covering it.

2. Method according to claim 1, characterised in that the coating (2) is provided with silica gel as an additional substance for the retention of water vapour, and that an atmospheric calibration is performed before impregnation of the coating (2) with a solvent (3).

3. Method according to claim 1, characterised in that the coating (2) is provided with sodium sulphite as the substance (1), and is impregnated with water as the solvent (3) for the formation of a zero-$O_2$-calibrating solution.

4. Method according to claim 1, characterised in that the coating (2) is provided with sodium sulphite from a sodium sulphite solution which is 70% to 100% saturated.

5. Method according to claim 1, characterised in that the coating (2) is provided with a substance (1) containing carbonate, and is impregnated with water as the solvent (3) to form a $CO_2$-calibrating solution of known $CO_2$ concentration.

6. Coating for performing a calibration of a measuring apparatus (9) for determining gas concentrations, in particular for the transcutaneous measuring of gases present in the blood, which is provided with a substance (1) for the delivery of a known concentration of the gas which is to be identified, and is arranged over the measuring face (4) of the measuring apparatus (9), characterised in that a porous strip (2) is provided with soluble substances (1), and can be moistened with a solvent (3) which dissolves the substances (1), in order to supply the known concentration of the gas to be determined.

7. Coating according to claim 6, characterised in that the strip (2) is partly provided with a covering (6).

8. Coating according to claim 6, characterised in that the strip (2) has several adjacent layers (7, 8) which are provided with different substances (1).

9. Coating according to claim 8, characterised in that a first layer (7) is provided with sodium sulphite for supplying a zero-$O_2$-concentration, and a second layer (8) is provided with silica gel for the retention of water vapour from the surrounding atmosphere and for supplying an atmospheric oxygen-concentration.

10. Coating according to claim 6, characterised in that the strip (2) is divided into several zones (10, 11) which are provided with different substances (1).

11. Coating according to claim 10, characterised in that a first zone (10) is provided with sodium sulphite for the formation of a zero-calibrating solution, and a second zone (11) is provided with a substance (1) containing carbonate for the formation of a known $CO_2$ concentration.

12. Coating according to claim 10, characterised in that the zones (10, 11) are separated from each other in each case by an intermediate piece (12).

13. Coating according to one of the claims 6 to 12, characterised in that the strip (2) has a thickness of approximately 0.2 mm to 1 mm.

1/1

10          6          2

Fig. 1

7    3          6          7

8    1    12    1    8

Fig. 2

10          2    12

9    4    5

Fig. 3

7